Europäisches Patentamt

European Patent Office

Office européen des brevets

(19)

(11) **EP 1 541 681 A1**

(12) **EUROPEAN PATENT APPLICATION**
published in accordance with Art. 158(3) EPC

(43) Date of publication:
**15.06.2005 Bulletin 2005/24**

(21) Application number: **03741480.2**

(22) Date of filing: **18.07.2003**

(51) Int Cl.⁷: **C12N 15/09**, C12N 15/86,
C12N 5/10, A01K 67/027,
A01H 5/00
// C12N5:10, C12R1:91

(86) International application number:
**PCT/JP2003/009155**

(87) International publication number:
**WO 2004/009813 (29.01.2004 Gazette 2004/05)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **18.07.2002 JP 2002209800**

(71) Applicant: **Mitsubishi Chemical Corporation
Tokyo 108-0014 (JP)**

(72) Inventors:
 • **KATSUKI, Motoya
 Minato-ku, Tokyo 106-0032 (JP)**
 • **Ishida, Mitsuyoshi
 Machida-shi, Tokyo 194-8511 (JP)**
 • **Morishita, Takeharu
 Machida-shi, Tokyo 194-8511 (JP)**

(74) Representative:
**Sternagel, Fleischer, Godemeyer & Partner
Patentanwälte,
An den Gärten 7
51491 Overath (DE)**

(54) **METHOD OF CONSTRUCTING NONHUMAN MAMMAL HAVING RNAi PHENOTYPE WITH THE USE OF PAPILOMAVIRUS VECTOR**

(57) It is an object of the present invention to improve a method for introducing dsRNA, so as to improve the efficiency of obtaining mammals such as mice having an RNAi phenotype. The present invention provides a method for producing a non-human mammal wherein the function of a target gene is suppressed, which comprises introducing into cells a papilloma virus vector into which the target gene has been incorporated.

EP 1 541 681 A1

Printed by Jouve, 75001 PARIS (FR)

**Description**

TECHNICAL FIELD

The present invention relates to a method for producing a non-human mammal having an RNAi phenotype using a papilloma virus vector.

BACKGROUND ART

To date, in order to clarify the functions of DNA at an individual level, a method comprising producing a gene knockout animal and analyzing the phenotype thereof has been applied. However, such a knockout method requires enormous efforts and time, and thus, it is not practical for analysis of a large number of gene functions. Accordingly, it is desired that a method for suppressing gene functions at an individual animal level, which is more effective and simple than the conventional knockout method will be developed.

The term "RNAi (RNA interference)" is used to mean a phenomenon whereby after RNA (double stranded RNA: dsRNA) formed by converting a part of mRNA encoding a part of a certain gene (referred to as a target gene) into a double stand has been introduced into a cell, the expression of the target gene is suppressed. In 1998, the fact that introduction of dsRNA into a living body exhibits action to suppress the expression of the same gene as the introduced gene was discovered in nematodes (Nature, 391 (6669) 806-811, 1998). Thereafter, such action has also been found in Eumycetes, plants *Nicotiana tabaccum* and *Oryza sativa*, planarias, *Trypanosoma brucei* (J. Biol. Chem., 275 (51) 40174-40179, 2000), the fly *Drosophila melanogaster* (Cell, 95 (7) 1017-1026, 1998), and zebra fish as a vertebrate animal. Thus, it has been considered that RNAi is a phenomenon, which is universally observed regardless of species.

The technical application of RNAi has been established in nematodes as a gene knockout technique. It has been utilized as a principal means for analyzing genome function using the total nucleotide sequence information obtained by a project for determining the total genomic sequence of a nematode (Nature, 408 (6810) 325-330, 2000; Nature, 408 (6810) 331-336, 2000). In the analysis of genome function of mammals also, RNAi is expected to be a method for efficiently suppressing gene expression, which is less burdensome than the gene knockout method in terms of a period of time and manpower.

With regard to mammals, the RNAi effect has been reported for the first time from an experiment wherein dsRNA was injected into an early embryo of a mouse (Nat. Cell Biol., 2 (2) 70-75, 2000). However, such an RNAi effect was observed only in the early embryo, and the RNAi effect disappeared when the mouse was born. It is considered that the reason why the RNAi effect disappeared is that dsRNA that had been introduced into a fertilized egg that is one-cell embryo was then diluted depending on the division and growth of the embryo, and that it could not maintain a concentration that is necessary for RNAi. In addition, there is also a possibility that mammals have mechanisms that biologically differ from those of nematodes.

To date, for the purpose of maintaining the intracellular concentration of dsRNA introduced, the present inventors have constructed a gene by ligating a gene comprising an inverted repeat sequence downstream of a mammalian expression vector and have introduced the constructed gene into a fertilized egg of an EGFP transgenic mouse. Thereafter, they have transferred the embryo into the oviduct of a mouse, so as to produce an EGFP dsRNA expression vector gene-introduced mouse. In the thus produced mice, several individuals having a phenotype in which the expression of a target gene (EGFP) was suppressed were observed (Japanese Patent Application No. 2001-46089). However, the efficiency of obtaining such mice was low. Thus, in order to analyze gene functions of an individual mouse using the RNAi effect, it has been desired that further improvements will be made.

DISCLOSURE OF THE INVENTION

It is an object of the present invention to solve the aforementioned problems of the prior art methods. In other words, it is an object of the present invention to improve a method for introducing dsRNA, so as to improve the efficiency of obtaining mammals such as mice having an RNAi phenotype.

The present inventors have conducted intensive studies directed towards achieving the aforementioned object. First, the present inventors introduced a BPV vector, into which an inverted repeat sequence had been introduced, into a fertilized egg, so as to produce a transgenic mouse. Thereafter, they obtained lymphocytes from the thus produced mouse and analyzed EGFP fluorescence of the lymphocytes with FACS. As a result, RNAi phenotypes were confirmed at a high efficiency such as approximately 30% of the number of individuals that were born. As described above, in order to avoid the dilution of the introduced DNA caused by cell division occurring in an early embryo, the present inventors used a BPV vector which is capable of replicating as a plasmid while synchronizing with the cell cycle, and dsRNA was allowed to express outside the chromosome. As a result, the present inventor has found that the RNAi effect is enhanced in an early embryo and non-human mammals having an RNAi phenotype can efficiently be obtained. The present invention has been completed based on these findings.

That is to say, the present invention provides a method for producing a non-human mammal wherein the function of a target gene is suppressed, which comprises introducing into cells a papilloma virus vector into which the target gene has been incorporated.

A papilloma virus vector into which the inverted repeat sequence of the target gene has been introduced is preferably used.

The papilloma virus is preferably a bovine papilloma virus vector.

Preferably, a papilloma virus vector into which a gene of interest has been incorporated is introduced into a fertilized egg, and a transgenic non-human mammal is produced by using the fertilized egg.

In another aspect, the present invention provides a non-human mammal wherein the function of a target gene is suppressed, which is produced by the aforementioned method of the present invention, a progeny thereof, or a portion thereof.

In another aspect, the present invention provides a recombinant vector formed by incorporating into a papilloma virus vector the inverted repeat sequence of a target gene capable of expressing in mammalian cells.

With regard to the recombinant vector of the present invention, the papilloma virus vector is preferably a bovine papilloma virus vector.

The recombinant vector of the present invention preferably comprises the inverted repeat sequence of a target gene downstream of a promoter sequence capable of functioning in mammalian cells.

In the recombinant vector of the present invention, the target gene is preferably a gene of a foreign reporter protein or a mutant protein thereof. Such a foreign reporter protein is preferably Enhanced green fluorescent protein (EGFP).

The recombinant vector of the present invention can be used to produce a transgenic non-human mammal.

In another aspect, the present invention provides a transformant having the aforementioned recombinant vector of the present invention.

In another aspect, the present invention provides an embryo generated from a fertilized egg derived from a non-human mammal into which the recombinant vector of the present invention has been introduced.

In another aspect, the present invention provides a fetus obtained by transplanting the aforementioned embryo into the uterus or oviduct of the corresponding non-human mammal, followed by development

BRIEF DESCRIPTION OF THE DRAWINGS

Figure 1 shows the overview of the construction of BPV expression vectors (BPV-EGFP IR and BPV-HIR constructs) comprising an EGFP IR (inverted repeat) DNA sequence.

Figure 2 shows fluorescence observation performed on individual mice. That is, baby mice that were born by natural childbirth were placed in a line, and fluorescence observation was performed.

Figure 3 shows the results of FACScan analysis performed on individual mice (BPV-HIR). Those having a ratio of reduction in fluorescence between 10% and 20% were evaluated as $\pm$.

Figure 4 shows the results of FACScan analysis performed on individual mice (BPV-EGFP IR). Those having a ratio of reduction in fluorescence between 10% and 20% were evaluated as $\pm$.

Figure 5 shows the results of fluorescence observation of each organ of an EGFP fluorescence-reduced mouse. Sample were prepared from a fluorescence reduced-mouse BPV-HIR106I (which was approximately 13 weeks old), and fluorescence in each organ thereof was observed.

Figure 6 shows the results of fluorescence observation of each organ of an EGFP fluorescence-reduced mouse. Samples were prepared from a fluorescence reduced-mouse BPV-HIR124I (which was approximately 13 weeks old), and fluorescence in each organ thereof was observed.

Figure 7 shows the results obtained by analyzing EGFP RNA expression in each organ of the EGFP fluorescence-reduced mice.

Lanes 1 to 4: Controls (EGFP)
Lane 5: BPV-HIR67I
Lane 6: BPV-HIR74I
Lane 7: BPV-HIR106I
Lane 8: BPV-HIR124I

BEST MODE FOR CARRYING OUT THE INVENTION

The embodiments of the present invention will be described in detail below.

The present invention relates to a method for producing a non-human mammal wherein the function of a target gene is suppressed, which comprises introducing into cells a papilloma virus vector into which the target gene has been incorporated.

From the studies that the present inventors have conducted to date, there was obtained a non-transgenic mouse having an RNAi phenotype in which the expression of a target gene is suppressed. In addition, based on the fact that the RNAi effect were confirmed in an ES cell line (Japanese Patent Application No. 2001-348705) and in an early embryo (Nat. Cell Biol., 2 (2) 70-75, 2000), it was considered that the RNAi effect in an early embryo is important for maintaining the RNAi effect in an individual mouse. Thus, it is thought that mice having an RNAi phenotype can efficiently be obtained by allowing an early embryo to have sufficient RNAi activity. Hence, a BPV (bovine papilloma virus) vector, which is effective for a large amount of gene expression in cytoplasms of non-human mammalian cells and enables transient expression, was used.

A BPV (bovine papilloma virus) vector (BCMGSNeo) (J. Exp. Med. 172, 969-972, 1990) has a size of approximately 14.3 kbp, and comprises a gene fragment *Hind*III + *Bam*HI (69% region) of BPV type 1, a part of a human β globin gene, a neomycin resistance gene, a CMV (Cytomegalovirus) promoter, the intron sequence and cDNA-introducing site of a rabbit β globin gene, and a rabbit β globin poly(A) signal. The BPV type 1 gene is a DNA virus, which infects the skin of a bovine to develop papilloma thereon. The gene is a 7,945 bp double-stranded cyclic DNA virus. The 69% region used for the above vector does not comprise sequences such as the sequence of a capsid protein associated with the infection of the above gene, but only comprises a region associated with replication. Thus, it maintains a character in which the BPV vector can replicate itself while synchronizing the cell cycle of non-human mammalian cells (*Tanpakushitsu, Kakusan, Koso* (Proteins, Nucleic acids, and enzymes), Vol. 40, No. 17, 2539-2544, 1995). In addition, it has been reported that the replication ability of the BPV vector is enhanced by introducing a part of a human β globin gene (Eur. J. Immunol., vol.18, 97-104, 1988).

In the present invention, a CMV enhancer, an EF1$\alpha$ promoter, the inverted repeat sequence of EGFP, and a poly(A) signal sequence derived from SV40 were introduced into the aforementioned BPV vector, so as to produce a plasmid, which allows the inverted repeat sequence of EGFP to express in non-human mammalian cells. This plasmid was then introduced into a mouse early embryo, so that the inverted repeat sequence was allowed to transiently express in the early embryo. Moreover, lymphocytes were obtained from the thus produced mouse, and using the lymphocytes, EGFP fluorescence was analyzed with FACS. As a result, it was found that approximately 30% of individuals born have an RNAi phenotype.

As a papilloma virus vector used in the present invention, the aforementioned BPV (bovine papilloma virus) vector (BCMGSNeo) (J. Exp. Med. 172, 969-972, 1990) can be used. However, vectors derived from papilloma viruses other than the above virus can also be used. Papilloma virus is a small DNA oncogenic virus, which belongs to Papovaviridae and has double-stranded cyclic DNA with a size of approximately 8 kb in the genome thereof. Various types of papilloma viruses such as a human papilloma virus, a rabbit papilloma virus, and a bovine papilloma virus have been known. Of these, bovine papilloma virus type 1 (BPV type 1) is commonly used as a cloning vector.

The term "inverted repeat sequence" is used to mean a sequence formed by placing a target gene and an inverted sequence thereof in order via a suitable sequence. More specifically, when a target gene has a double-strand consisting of an n number of nucleotide sequences indicated below:

$$5'\text{-}X_1X_2 \text{ ...... } X_{n-1}X_n\text{-}3'$$

$$3'\text{-}Y_1Y_2 \text{ ...... } Y_{n-1}Y_n\text{-}5'$$

the inverted sequence thereof has the following sequences:

$$5'\text{-}Y_nY_{n-1} \text{ ...... } Y_2Y_1\text{-}3'$$

$$3'\text{-}X_nX_{n-1} \text{ ...... } X_2X_1\text{-}5'$$

wherein, with regard to the nucleotide represented by X and the nucleotide represented by Y, those having the same numerical script are nucleotides complementary to one another.

The inverted repeat sequence is a sequence formed by placing the aforementioned two types of sequences in order via a suitable sequence. Regarding such inverted repeat sequences, there are two cases: a case where the sequence of a target gene is located upstream of such an inverted sequence; and a case where such an inverted sequence is located upstream of the sequence of a target gene. Both of the above inverted repeat sequences may be used in the present invention. Preferably, an inverted sequence is located upstream of the sequence of a target gene.

A sequence existing between the sequence of a target gene and the inverted sequence thereof is a region that forms a hairpin loop when it is transcribed into RNA. The length of this region is not particularly limited, as long as it can form a hairpin loop. It is generally between 0 bp and 700 bp, preferably between 0 bp and 300 bp, and more preferably between 0 bp and 100 bp. A restriction site may exist in this sequence.

Any given gene can be used as a target gene in the present invention. When a transgenic animal is produced by using the recombinant vector of the present invention and gene knockout is then intended by the RNAi effect, the target gene is a gene the expression of which is intended to be suppressed (a gene intended to be knockout). Such target genes also include genes, which have been cloned but the functions of which are still unknown.

Otherwise, such a target gene may also be a gene of a foreign reporter protein or a mutant protein thereof. When a gene of a foreign reporter protein gene or its mutant protein is used as a target gene, the RNAi effect can easily be detected and evaluated by a transgenic technique of using the recombinant vector of the present invention

Examples of a foreign reporter protein may include Enhanced green fluorescent protein, Green fluorescent protein, aequorin, chloramphenicol acetyltransferase, β-galactosidase, luciferase, and β-glucuronidase.

An example of a mutant protein of such a foreign reporter protein may be a protein, which comprises a substitution, deletion, addition, and/or insertion of one or several (for example, 1 to 20, preferably 1 to 10, and more preferably 1 to 5) amino acids with respect to the amino acid sequence of the above-described wild type reporter protein, and which preferably maintains functions equivalent to or greater than those of the wild type reporter protein.

Specific examples of the gene of such a mutant reporter protein used herein may include a gene comprising a deletion of a part of the nucleotide sequence of a reporter protein gene, a gene comprising a substitution of the nucleotide sequence of a reporter gene with another nucleotide sequence, and a gene comprising an insertion of another nucleotide sequence into a part of the nucleotide sequence of a reporter gene. The number of nucleotides to be deleted, substituted, or added, is not particularly limited. It is generally between 1 and 60, preferably between 1 and 30, and more preferably between 1 and 10. These mutant genes desirably maintain the functions of reporter genes.

A gene of a mutant protein can be produced by any given methods that have already been known to a person skilled in the art, such as chemical synthesis, genetic engineering, or mutagenesis. Specifically, an agent acting as a mutagene is allowed to contact with DNA encoding a natural reporter protein so as to allow the agent to act thereon, or ultraviolet ray is applied. Otherwise, genetic engineering such as the PCR method is used, thereby obtaining a gene encoding a mutant protein. Site-directed mutagenesis which is a genetic engineering method, is particularly effective in that it is a method for introducing a specific mutation into a specific site. Such site-directed mutagenesis can be applied by methods described in Molecular Cloning: A laboratory Manual, 2nd ED., Cold Spring Harbor Laboratory, Cold Spring Harbor, NY., 1989; and Current Protocols in Molecular Biology, Supplement 1 to 38, John Wiley & Sons (1987-1997).

In the recombinant vector of the present invention which is produced by using papilloma virus, the inverted repeat sequence of a target gene exists downstream of a promoter sequence capable of functioning in mammals. With such a structure, it becomes possible to allow the inverted repeat sequence of the target gene to express in mammalian cells. That is to say, in the recombinant vector of the present invention, the inverted repeat sequence of a target gene is located such that it is under the control of the aforementioned promoter.

A promoter sequence used in the present invention is not particularly limited, as long as it can function in mammals.

Examples of such a promoter capable of expressing in non-human animals may include gene promoters derived from viruses (e.g. Cytomegalovirus, Moloney leukemia virus, JC virus, mammary tumor virus, etc.), and promoters derived from various types of mammals (e.g. humans, rabbits, dogs, cats, Guinea pigs, hamsters, rats, mice, etc.). Examples of promoters derived from various types of mammals may include promoters derived from albumin, endothelin, osteocalcin, muscle creatine kinase, collagen type I and II, cyclic AMP-dependent protein kinase β subunit (The Journal of Biological Chemistry, Vol. 271, No. 3, pp. 1638-1644, 1996), atrial natriuretic factor, dopamine β-hydroxylase, neurofilament light chain (The Journal of Biological Chemistry, Vol. 270, No. 43, pp. 25739-25745, 1995; and the same publication, Vol. 272, No. 40, pp. 25112-25120, 1997), metallothionein, metalloproteinase 1 tissue inhibitor, smooth muscle α-actin, polypeptide chain elongation factor 1α (EF-1α), β-actin, α- and β-myosin heavy chain, myosin light chain 1 and 2, myelin basic protein, serum amyloid P component, or renin.

Other than the aforementioned examples, there can also be used promoters described in Manual Disease-Model Mice, Molecular Medicine, Extra Edition, edited by Kenichi Yamamura, Motoya Katsuki, and Shinichi Aizawa, Nakayama Shoten Co., Ltd.

An EF1α promoter used in the examples in the present specification is preferably used in the present invention.

Other than this promoter, the following promoters are also preferably used in the present invention.

(1) β-actin promoter

In general, a β-actin promoter is used in combination with a CMV enhancer. Examples may include pCAGGS, a chicken beta-actin promoter and a cytomegalo virus enhancer, and beta-actin intron and bovine globin poly-adenylation signal. This is described in a cited reference, H. Niwa, K. Yamanami, J. Miyazaki, Gene, 108, (1991) 193-199.

(2) CMV promoter

In general, a CMV promoter is used in combination with a CMV enhancer. This is described in a cited reference, Janet A. Sawicki et al., Experimental Cell Research 244, 367-369 (1998).

(3) Metallothionein promoter

Please refer to Establishment of Transgenic Mice Carrying Human Fetus-Specific CYP3A7, Yong Li et al, Archives of Biochemistry and Biophysics, Vol. 329, No. 2, 235-240, 1996.

(4) Apolipoprotein E promoter

Apolipoprotein E promoter is a promoter that is intended to be expressed in fetal liver. This is described in Simonet et al., 1993, J. Biol. Chem., 268, 8221-8229.

(5) Promoter of a gene itself that is intended to be introduced

This case includes introduction of the genome itself to produce a transgenic mouse. This is described in Okamoto M. et al., J. Exp. Med., 175, 71 (1992).

The recombinant vector of the present invention may comprise an enhancer sequence upstream of a promoter sequence. The above CMV enhancer is an example of an enhancer sequence used herein.

The recombinant gene of the present invention may comprise an insulator sequence or a part thereof. The term "insulator sequence" is used to mean a gene sequence that prevents the suppression of gene expression caused by the "position effect" in transgenic animals. The insulator sequence is expected to act as a barrier to the influence of neighboring cis-elements.

The location of such an insulator sequence or a part thereof is not particularly limited. In terms of its effects, however, it is preferably located on the 5'-side (upstream) of an introduced gene (that is, the inverted repeat sequence of a target gene). Most preferably, an insulator sequence or a part thereof is located upstream of a promoter sequence (or when an enhancer sequence exists, it is located upstream of the enhancer sequence).

Other than a chicken β-globin-derived insulator sequence described in the examples of the present specification, examples of an insulator sequence that can be used in the present invention may include the following sequences, but examples are not limited thereto.

(1) Fruit-fly scs and scs' sequence
Rebecca Kellum and Paul Schedl, Cell, Vol. 64, 941-950, March 8, 1991
(2) Fruit-fly gypsy transposon insulator sequence
Holdrige, C., and D. Dorsett, 1991 Mol. Cell. Biol. 11: 1894-1900
(3) Sea urchin arylsulfatase insulator sequence
Koji Akasaka et. al., Cellular and Molecular Biology 45 ( 5 ), 555-565, 1999
(4) Human T cell receptor α/δ locus BEAD element
Zhong, X. P., and M. S. Krangel, 1997, Proc. Natul. Acad. Sci. U.S.A.
(5) Human apolipoprotein B-100 (apoB) matrix attachment site
Namciu et al, 1998, Mol. Cell. Biol. 18: 2382-2391

The recombinant vector of the present invention may comprise a poly(A) addition signal sequence downstream of the inverted repeat sequence of a target gene. By inserting the poly(A) addition signal sequence, transcription of messenger RNA of interest can be terminated.

A specific example of such a poly(A) addition signal sequence may include an SV40 poly(A) addition signal, but examples are not limited thereto.

The recombinant vector of the present invention can be produced by known methods or methods equivalent thereto.

The present invention further relates to a transformant having the aforementioned recombinant vector of the present invention.

When a bacterium is used as a host, specific examples of a vector may include pBTrP2, pBTac1 and pBTac2 (both of which are commercially available from Boehringer Mannheim), pKK233-2 (manufactured by Pharmacia), pSE280 (manufactured by Invitrogen), pGEMEX-1 (manufactured by Promega), pQE-8 (manufactured by QIAGEN), pQE-30 (manufactured by QIAGEN), pKYP10 (Japanese Patent Application Laid-Open (Kokai) No. 58-110600), pKYP200 [Agrc. Biol. Chem., 48, 669 (1984)], PLSA1 [Agrc. Blo1. Chem., 53, 277 (1989)], pGEL1 [Proc. Natl. Acad. Sci. USA, 82, 4306 (1985)], pBluescrlptII SK+, pBluescriptII SK(-) (manufactured by Stratagene), pTrS30 (FERM BP-5407), pTrS32 (FERM BP-5408), pGEX (manufactured by Pharmacia), pET-3 (manufactured by Novagen), pTerm2 (US4686191, US4939094, US5160735), pSupex, pUB110, pTP5, pC194, pUC18 [Gene, 33, 103 (1985)], pUC19 [Gene, 33, 103 (1985)], pSTV28 (manufactured by Takara Shuzo Co., Ltd.), pSTV29 (manufactured by Takara Shuzo Co., Ltd.), pUC118 (manufactured by Takara Shuzo Co., Ltd.), pPA1 (Japanese Patent Application Laid-Open (Kokai) No. 63-233798), pEG400 [J. Bacteriol., 172, 2392 (1990)], and pQE-30 (manufactured by QIAGEN), but examples are not limited thereto.

When yeast is used as a host, specific examples of a vector may include YEp13 (ATCC37115), YEp24 (ATCC37051), Ycp50(ATCC37419), pHS19, and pHS15, but examples are not limited thereto.

When an animal cell is used as a host, specific examples of a vector may include pcDNAI, pcDM8 (commercially available from Funakoshi), pAGE107 [Japanese Patent Application Laid-Open (Kokai) No. 3-22979; Cytotechnology, 3, 133, (1990)], pAS3-3 (Japanese Patent Application Laid-Open (Kokai) No. 2-227075), pCDM8 [Nature, 329, 840, (1987)], pcDNAI/AmP (manufactured by Invitrogen), pREP4 (manufactured by Invitrogen), pAGE103 [J.Blochem., 101, 1307 (1987)], and pAGE210, but examples are not limited thereto.

As a host cell used to produce a transformant, any type of host cell can be used, as long as it allows a gene of interest to express therein. Examples of such a host cell may include bacteria (e.g. Escherichia, Serratia, Corynebacterium, Brevibacterium, Pseudomonas, Bacillus, microbacterium, etc.), yeasts (e.g. Kluyveromyces, Saccharomyces, Schizosaccharomyces, Trichosporon, Schwan-niomyces, etc.), animal cells (e.g. Namalva cells, COS1 cells, COS7 cells, CHO cells, etc.), plant cells, and insect cells (e.g. Sf9 cells, Sf21 cells, High5 cells, etc.).

A method for introducing a recombinant vector into a host can appropriately be selected depending on the type of the host or the like. Examples of a method for introducing a recombinant vector into a bacterium host may include a method of using calcium ion and the protoplast method. Examples of a method for introducing a recombinant vector into a yeast host may include electroporation, the spheroplast method, and the lithium acetate method. Examples of a method for introducing a recombinant vector into an animal cell include electroporation, the calcium phosphate method, and lipofection.

The present invention further relates to: an embryo developed from a fertilized egg derived from a non-human mammal into which the aforementioned recombinant vector of the present invention has been introduced; and a fetus obtained by transplanting this embryo into the uterus or oviduct of the corresponding non-human mammal, followed by development. These animals are transgenic non-human mammals, which allow the inverted repeat sequence of a target gene to express.

Examples of a part of the above non-human mammal may include the cell organella, cell, tissue, and organ of the non-human mammal, as well as the head, finger, hand, foot, abdomen, and tail thereof.

Examples of a non-human mammal may include a mouse, a rat, a hamster, a Guinea pig, a rabbit, a dog, a cat, a horse, a bovine, a sheep, a swine, a goat, and a monkey, but examples are not limited thereto. As such a non-human mammal, rodents such as a mouse, rat, or Guinea pig are preferable, and a mouse and a rat are particularly preferable. Examples of a mouse may include inbred mice such as C57BL/6 or DBA2, and hybrid mice such as B6C3F1, BDF1, B6D2F1, BALB/c, or ICR. Specific examples of a rat may include Wistar and SD.

In a preferred embodiment, the transgenic non-human mammal of the present invention may have DNA into which the inverted repeat sequence of a target gene has been incorporated such that it can be expressed at a specific site.

The expression "such that the inverted repeat sequence of a target gene can be expressed at a specific site" is used to mean that the inverted repeat sequence of a target gene can be expressed at a specific intracellular site, that is, a specific site such as a cell organella, cell, tissue, or organ.

An example of such an intracellular specific site may be an axis cylinder located in a nerve cell or the like. Examples of a cell organella may include a nucleus, a mitochondrion, a Golgi apparatus, an endoplasmic reticulum, a ribosome, and a cell membrane. Examples of a cell may include mammals' hepatic cell, splenic cell, nerve cell, glial cell, pancreatic β cell, bone marrow cell, mesangial cell, Langerhans cell, epidermal cell, epidermic cell, endothelial cell, fibroblast, fibrocyte, muscle cell, fat cell, immunocyte, megakaryocyte, synovial cell, chondrocyte, osteocyte, osteoblast, osteoclast, mammary glandular cell, hepatic cell or interstitial cell, or the precursor cell, stem cell, or cancer cell thereof. Examples of a tissue may include any types of tissues where the aforementioned cells exist, such as the brain (amygdaloid nucleus, basal ganglia, hippocampus, hypothalamus, cerebral cortex, medulla oblongata, cerebellum, pineal body, etc.), spinal cord, pituitary gland, stomach, gonad, thyroid gland, gallbladder, bone marrow, adrenal gland, skin, muscle, lung, large intestine, small intestine, duodenum, rectum, blood vessel, thymus gland, submaxillary salivary gland, peripheral blood, prostate, testis, ovary, placenta, uterus, bone, articulation, or skeletal muscle. Examples of such a tissue may further include blood cells and the cultured cells of the aforementioned cells. Examples of an organ may include the heart, kidney, pancreas, liver, and spleen.

In one of the preferred embodiments, the transgenic non-human mammal of the present invention may have DNA into which the inverted repeat sequence of a target gene has been incorporated such that it can be expressed in a specific period.

The term "in a specific period" is used in the present specification to mean a specific period ranging from the generation of an embryo, birth, or development, to death. Thus, such a specific period may be each stage in the generation of an embryo, including the period when a foreign gene was introduced, or may be any period after a certain period of time has passed, such as a period of time by the hour, by the day, by the week, by the month, or by the year.

In order to allow the inverted repeat sequence of a target gene to express in a specific period in the transgenic non-human mammal of the present invention, using an expression vector into which a promoter region capable of allowing a protein to express in a specific period, a signal sequence capable of allowing a protein to express in a specific period, or the like has been incorporated, a transgenic non-human mammal having the inverted repeat sequence of the target gene is produced.

The inverted repeat sequence of a target gene can also be allowed to express in a specific period by constructing a protein expression inducible system or by administering a protein expression inducible agent to a non-human mammal in a specific period. An example of such a protein expression inducible system may be an inducible expression system in which tetracycline or ecdysone is used. Those administered in such a system may include tetracycline or an analog thereof, and ecdysone or an analog thereof. In addition, a cre-lox P system using recombinase may also be used.

Moreover, the inverted repeat sequence of a target gene is also allowed to express in a specific period by incorporating a gene resistant to antibiotics such as tetracycline, kanamycin, hygromycin, or puromycin into an expression vector. The location of the aforementioned resistance gene in the expression vector of the present invention is not particularly limited. In general, such a resistance gene is preferably located downstream of a reporter gene or mutant gene thereof, or upstream of a promoter region or signal sequence.

The transgenic non-human mammal of the present invention can be produced by introducing a recombinant vector (hereinafter referred to as an "transgene" at times) comprising the inverted repeat sequence of a target gene downstream of a promoter sequence capable of functioning in mammalian cells, into a target animal. Specifically, such a transgene is introduced into the fertilized egg, embryonic stem cell, somatic cell, sperm, or unfertilized egg of a non-human mammal that is a target, so as to obtain a transgenic non-human mammal, on the chromosomes of all cells including germinative cells of which the above gene has been incorporated. Introduction of a transgene into the fertilized egg, embryonic stem cell, somatic cell, sperm, or unfertilized egg should be carried out, such that the trangene exists on the chromosomes of all cells including germinative cells and somatic cells of a target non-human mammal.

Progenies of the aforementioned transgenic non-human mammal, on the chromosomes of all cells including germinative cells of which the above gene has been incorporated, also have the above gene. Homozygous animals having such transgene on both of the homologous chromosomes thereof are obtained, and a female homozygous animal is bred with a male homozygous animal, so as to produce progenies. It is then confirmed that all of the thus obtained progenies stably maintain or have the above gene, and thereafter, breeding and subculture can be carried out in an ordinary breeding environment.

A method for producing the transgenic non-human mammal of the present invention will be described further in detail below.

The transgenic non-human mammal of the present invention can be produced by introducing a transgene into germ cells and the like, including a fertilized egg, an unfertilized egg, sperm, and an initial cell thereof, preferably at an early stage of the formation of embryo in the development of a non-human mammal (more preferably at a stage of a single cell or amphicytula, which is generally before the 8-cell-stage).

The structure of the transgene and the construction method thereof are as described above in the present specification.

When a transgene is introduced into the fertilized egg of a non-human mammal as a target or a progenitor thereof, the used fertilized egg is obtained by breeding a male non-human mammal with a female non-human mammal of the same type. Such a fertilized egg can be obtained by natural crossbreeding, but it is preferable that the sexual cycle of the female non-human mammal be artificially controlled and then bred with the male non-human mammal. As a method for artificially controlling the sexual cycle of a female non-human mammal, it is preferable that follicle-stimulating hormone (pregnant mare serum gonadotrophin, PMSG) be first administered to the abdominal cavity of the animal by injection and that luteinizing hormone (human chorionic gonadotropin, hCG) be then administered thereto by injection. Preferred dosage and administration interval of such hormones can be determined as appropriate depending on the type of a non-human mammal.

In order to introduce such a transgene into a non-human mammal, known methods (for example, the calcium phosphate method, the electropulse method, lipofection, the agglutination method, microinjection, particle gun, the DEAE-dextran method, etc.) can be used. In addition, it is also possible that a transgene of interest be introduced into a somatic cell by the aforementioned introduction method and that the obtained cell (or the nucleus thereof) be allowed to be fused with the aforementioned germ cell by a known cell fusion method, so as to produce the transgenic non-human mammal of the present invention.

The structure of the transgene and the construction method thereof are as described above in the present specification. When a transgene is introduced into a non-human mammal that is at a stage of a fertilized egg, it is ensured that the transgene exists in all the germ cells and somatic cells of the target animal.

After the transgene has been introduced into the fertilized egg, it is artificially transplanted or implanted into a female non-human mammal. As a result, a transgenic non-human mammal having the transgene is obtained. A preferred method comprises administering luteinizing hormone-releasing hormone (LHRH) or an analog thereof to a female non-human mammal and then breeding the female non-human mammal with a male non-human mammal, so that a fertilized egg into which the transgene has been introduced can artificially be transplanted or implanted to the pseudopregnant female non-human mammal whose fertilization ability has been induced. The amount of LHRH or an analog thereof administered, and the period when a female non-human mammal is bred with a male non-human mammal after the administration of LHRH or an analog thereof, can be selected as appropriate depending on the type of non-human mammals or the like.

Whether or not the transgene has been incorporated into genomic DNA can be confirmed by extracting DNA from the tail of an animal born and examining it by Southern hybridization or the PCR method.

The fact that the transgene exists in the germ cells of an animal produced after introduction of the transgene means that progenies from the produced animal have such transgenes in all the germ cells and somatic cells. The progenies that inherited the transgene from the above animal have the same transgene in all the germ cells and somatic cells.

Homozygous animals having the transgene on both of the homologous chromosomes thereof are obtained, and a female homozygous animal is bred with a male homozygous animal, so as to produce progenies all of which have the above transgene. The thus obtained progenies are also included in the animal of the present invention.

The detailed explanations on production of a transgenic animal are described in, for example, Manipulating the Mouse Embryo (Brigid Hogan et al., Cold Spring Harbor Laboratory Press, 1986); Gene Targeting, A Practical Approach, IRL Press at Oxford University Press (1993); Bio Manual Series 8, Gene Targeting, Production of Mutant Mice Using ES Cells, Yodosha, Co., Ltd (1995); and Developmental Engineering Experiment Manual, Production Method of Transgenic Mice, Kodansha Ltd. (1987).

With regard to the transgenic non-human mammal of the present invention that expresses the inverted repeat sequence of a target gene, it is expected that the expression of the target gene is suppressed by the RNAi effect. That is to say, a method for suppressing the expression of a target gene, which comprises introducing into non-human mammalian cells a recombinant vector containing the inverted repeat sequence of the target gene downstream of an enhancer sequence and a promoter sequence, is also included in the scope of the present invention.

The aforementioned model animal wherein the functions of a target gene are "knockout" is useful for analysis of the functions of a novel gene and the like.

The present invention will be more specifically described in the following examples. However, the examples are not intended to limit the scope of the present invention.

EXAMPLES

Example 1: Construction of transgene 5' INS 240 CE EGFP IR

In the early studies of insulators, a transgene 5' INS 240 CE EGFP IR was produced from a vector having the same insulator sequences of 240 residues on both 5'- and 3'-sides of a cloning site. Thereafter, the results obtained from experiments with models suggested that the case where an insulator sequence is inserted only into the 5'-side of a transgene is the most effective. Accordingly, from pUC19 5'3' INS240 CE EGFP IR that has the same insulator sequences of 240 residues on both 5'- and 3'-sides and also has the inverted repeat sequence of EGFP downstream of a CMV enhancer and an EF1α promoter, the 3' insulator sequence inserted into the 3'-terminus of the transgene was eliminated, so as to construct a transgene 5' INS 240 CE EGFP IR, as mentioned follows.

The XhoI-AflII fragment of pCE-EGFP-1 (publication: Takada, T. et al, Selective production of transgenic mice using green fluorescent protein as a marker. Nature Biotech. 15: 458-461, 1997) was inserted into in two steps and ligated to the XhoI-AflII site of pUC19 5',3' INS240 (which was obtained by chemically synthesizing 10 fragments of a chicken β-globin-derived insulator sequence gene, and then inserting a fragment of 240 base pairs which was obtained by ligating these 10 fragments with DNA ligase, into the multicloning site of a pUC19 vector). Thereafter, *Escherichia coli* JM109 was transformed therewith, so as to obtain a plasmid pUC19 5',3' INS240 CE EGFP.

The *Kpn*I-*Xho*I fragment of Litmus28 EGFP was inserted into the *Kpn*I-*Sal*I cleavage site of the above pUC19 5',3' INS240 CE EGFP, and they were ligated to each other. Thereafter, *Escherichia coli* SURE2 (Stratagene) was transformed therewith, so as to obtain a plasmid pUC19 5',3' INS240 CE EGFP IR having an inverted repeat sequence.

A fragment containing the 3' insulator sequence was cleaved from pUC19 5',3' INS240 CE EGFP IR as follows.

pUC19 5',3' INS240 CE EGFP IR was treated with *Kpn*I and *Swa*I. A pUC19 5',3' INS240 CE EGFP IR/ *Kpn*I-*Swa*I vector was confirmed from a DNA fragment with a size of approximately 5.4 kbp obtained by electrophoresis. Then, a dephosphorylation treatment was carried out with BAP to prevent self-ligation. Thereafter, phenol extraction, chloroform extraction, and ethanol precipitation were carried out, and BAP was removed.

A plasmid pEGFP-1 SwL obtained by inserting a linker having a *Swa*I restriction site into the *Afl*II restriction site of pEGFP-1 (Clontech), was treated with *Kpn*I and *Swa*I, and the plasmid was then electrophoresed on agarose gel. A DNA fragment with a size of approximately 1.0 kbp was cut out, and it was defined as a *Kpn*I-*Swa*I fragment.

The pUC19 5',3' INS240 CE EGFP IR/*Kpn*I-*Swa*I vector was ligated to the *Kpn*I-*Swa*I fragment using DNA Ligation Kit ver. 2 (Takara). *Escherichia coli* SURE2 was transformed therewith, followed by screening with the length of a DNA fragment treated with *Eco*T22I, *Swa*I and *Not*I, so as to obtain a positive clone. After confirming the sequence of the DNA, it was named as pUC19 5' INS240 CE EGFP IR.

A plasmid pUC19 5' INS240 CE HIR having an EGFP dsDNA expression gene (inverted repeat sequence gene) was constructed by the following procedures.

Using pCE-EGFP-1 as a template, PCR was carried out with primers containing a restriction enzyme *Sfi*I site (SEQ ID NOS: 1 and 2) and primers containing a *Cpo*I site (SEQ ID NOS: 3 and 4), so as to obtain a DNA fragment containing the restriction enzyme *Sfi*I site and having a half-length of EGFP, or a DNA fragment containing the *Cpo*I site and having a half-length of EGFP.

Primers used in PCR

EGFPSfiI-A: GGCCATATAGGCCAGTTGTACTCCAGCTTGTGC (SEQ ID NO: 1)

EGFPSfiI-B: GGCCTACATGGCCTAAACGGCCACAAGTTCAGC (SEQ ID NO: 2)

EGFPCpoI-A: CGGACCGTAAACGGCCACAAGTTCAGC (SEQ ID NO: 3)

EGFPCpoI-B: CGGTCCGAGTTGTACTCCAGCTTGTGC (SEQ ID NO: 4)

PCR conditions consisted of 94°C, 5 minutes, (94°C, 30 seconds, 60°C, 30 seconds, and 72°C, 30 seconds) x 30 cycles, and 72°C, 10 minutes. The obtained DNA fragment with a half-length of EGFP was treated with *Cpo*I or *Sfi*I. After agarose gel electrophoresis, a DNA fragment was recovered. On the other hand, a pSC3 vector (FEBS Letters 479, 79-82, 2000) was treated with *Cpo*I. Agarose gel electrophoresis was carried out thereon, and then a DNA fragment was recovered. The DNA fragment-*Cpo*I with a half-length of EGFP was ligated to the pSC3 vector-*Cpo*I, and *Escherichia coli* JM109 was transformed therewith, so as to obtain a plasmid pSC3-EGFP-HL*Cpo*I. Subsequently, the pSC3-EGFP-HL*Cpo*I was treated with *Sfi*I and then electrophoresed on agarose gel, and a DNA fragment was recovered. Subsequently, the DNA fragment-*Sfi*I with a half-length of EGFP was ligated to the pSC3-EGFP-HL*Cpo*I-*Sfi*I, and *Escherichia coli* SURE2 (Stratagene) was transformed therewith, so as to obtain a plasmid pSC3-EGFP-HL-*Cpo*I-*Sfi*I having an inverted repeat sequence.

Thereafter, the plasmid pSC3-EGFP-HL*Cpo*I-*Sfi*I was treated with *Not*I and then electrophoresed on agarose gel, and a DNA fragment with a size of approximately 0.8 kbp was recovered. On the other hand, a pUC19 5' INS240 CEpA vector was treated with *Not*I, and then, a dephosphorylation treatment was carried out with BAP to prevent self-ligation. Thereafter, phenol extraction, chloroform extraction, and ethanol precipitation were carried out, and BAP was removed.

pSC3-EGFP-HL*Cpo*I-*Sfi*I-*Not*I was ligated to the pUC19 5' INS240 CEpA vector-*Not*I, and *Escherichia coli* SURE2 (Stratagene) was transformed therewith, so as to obtain a plasmid pUC19 5' INS240 CE HIR having an inverted repeat sequence.

Example 2: Construction of BPV expression vector containing EGFP IR (inverted repeat) DNA sequence

A BPV (bovine papilloma virus) vector (BCMGSNeo) (J. Exp. Med. 172, 969-972, 1990) was treated with *Xba*I and *Bam*HI, and a fragment containing a BPV 69% region was then cut out.

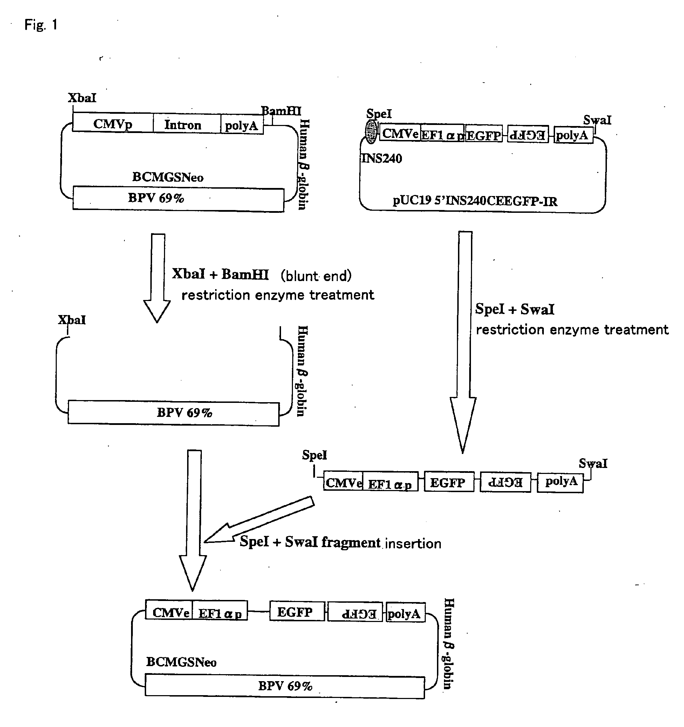

Subsequently, pUC19 5' INS240 CE EGFP IR (constructed in Example 1) and pUC19 5' INS240 CE HIR (constructed in Example 1) were treated with *Spe*I and *Swa*I. Thereafter, a fragment ranging from a Cytomegalovirus enhancer to a SV40 (a simian virus 40)-derived polyA tail including EGFP IR was cut out and subjected to a ligation reaction, so as to obtain a BPV-EGFP IR vector and a BPV-HIR vector, respectively (Figure 1).

Example 3: Preparation of BPV expression vector containing EGFP IR (inverted repeat) DNA sequence

Both the BPV-EGFP IR plasmid and the BPV-HIR plasmid were prepared in large scale by the alkali lysis method. Each of the thus prepared plasmids was further purified by ultracentrifugation and then diluted with PBS(-) resulting in a concentration of 1.5 ng/ml. The obtained solution was filtrated through a filter with a pore-size of 0.22 μm, so as to produce a plasmid to be introduced.

Example 4: Production of fertilized egg

Fertilized eggs were produced by *in vitro* fertilization according to the method of Toyoda et al. (Studies regarding *in vitro* fertilization of mouse eggs, Animal Breeding Magazine 16: 147-151, 1971). That is to say, PMGS and hCG (5 units) were injected into a female mouse intraperitoneally at an interval of 48 hours. 16 to 18 hours after the injection, eggs were collected and then inseminated with the seminal fluid (which was collected 1.5 hours before collection of the egg; approximately 100 to 150 sperms/μl) of an EGFP transgenic mouse (Masaru Okabe et al, FEBS Letters 407 (1997) 313-319). Approximately 6 hours after the insemination, the release of the secondary polar bodies of the eggs and the presence or absence of both male and female pronuclei were confirmed. Thereafter, only fertilized eggs were collected. The obtained-pronuclear eggs were cryopreserved by simple vitrification method according to the method of Nakao et al. (1997). The cryopreserved eggs were melted before undergoing experiments, and they were then subjected to microinjection.

Example 5: Microinjeciton of DNA

DNA was microinjected into the pronucleus of the fertilized egg according to the method of Katsuki et al. (Developmental Engineering Experiment Manual, Production Method of Transgenic Mice, 1987). The eggs were transferred into droplet of modified Whitten's medium (mWM). In the case of intranuclear injection, after a male pronucleus had been confirmed under a phase contrast microscope (Invert Scope D; Zeiss), approximately 2 pl of the purified DNA solution (1.5 ng/ul) was injected to the pronucleus. In the case of intracytoplasmic injection, approximately 2 pl of the DNA solution was injected into the cytoplasm. The survival embryo was transferred into the mWM embryo, and it was then cultured under conditions of 5%$CO_2$, 95% air, and 37°C. Thereafter, the embryo was transferred into the oviduct of a pseudopregnant female ICR mouse, and it was implanted.

Example 6: Typing of genotype

A 1 cm portion was cut out of the tail of a mouse at 4 weeks after the birth, and a a solubilizing buffer containing pronase K and proteinase E was added thereto. The obtained mixture was solubilized at 55°C overnight. Thereafter, DNA was obtained from the solubilized solution using DNA extractor (manufactured by Kurabo). The obtained DNA was analyzed by Southern hybridization using a DNA probe containing an EGFP gene sequence (that was labeled by the known radioisotope labeling method). A commercially available labeling kit (manufactured by Stratagene) was used for labeling. Approximately 10 μg of DNA was completely cleaved with restriction enzymes *Dra*I and *Bam*HI, and the obtained DNA fragment was electrophoresed on 1% agarose gel. It was then transferred on a nylon filter according to the method described in the Southern hybridization method (Journal Molecular Biology, vol. 98, p. 503, 1975). This filter was hybridized with a probe in a perfect hybridization solution (manufactured by Toyobo) for 2 hours. Thereafter, the resultant filter was washed with 2 x SSC and 0.1% SDS at 65°C for 5 minutes twice, and further washed therewith for 30 minutes twice. As a result of the Southern hybridization, with regard to BPV-HIR, it was confirmed that 8 out of the tested 156 individuals had the HIR gene. On the other hand, with regard to BPV-EGFP IR, it was confirmed that 2 out of the tested 71 individuals had the EGFP IR gene.

Example 7: Fluorescence observation in individual mouse

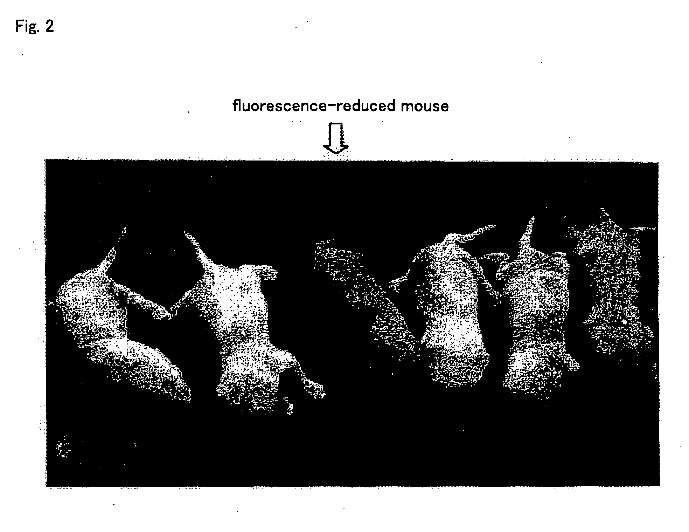

The aforementioned pseudopregnant female ICR mouse was subjected to caesarean section at 18 days after implantation of the embryo, so as to obtain baby mice. A 365 nm ultraviolet lamp (UVL-56; UVP) was applied to the obtained baby mice in a dark box (C-10; UVP), so as to carry out EGFP fluorescence observation. As a result, among baby mice produced from the fertilized eggs into which the EGFP dsRNA expression gene had been microinjected, several baby mice with reduced EGFP fluorescence were confirmed by observing their body surfaces (Figure 2; a safety goggle against ultraviolet light was used to eliminate blue haze generated by long wavelength ultraviolet light, and a digital camera was used for photographing.)

Example 8: Analysis of phenotype in individual mouse

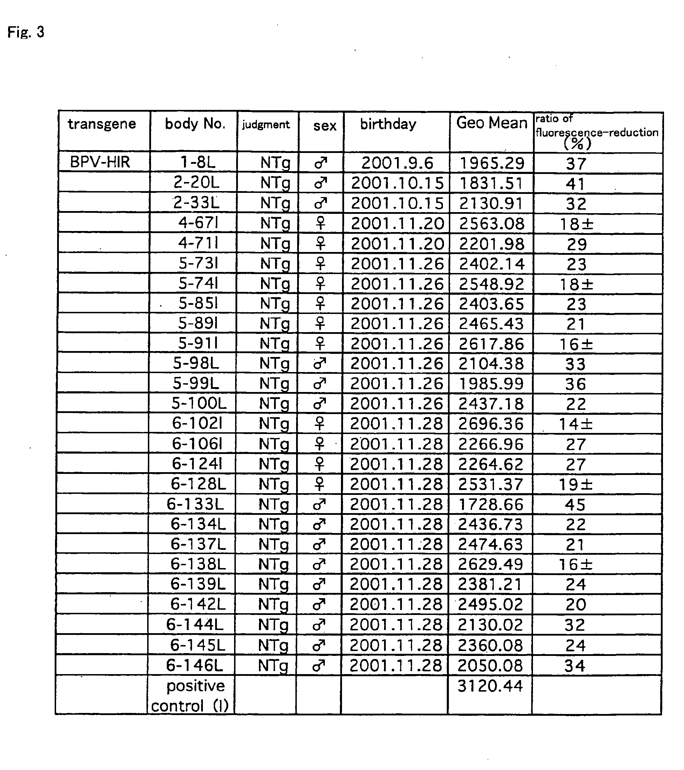

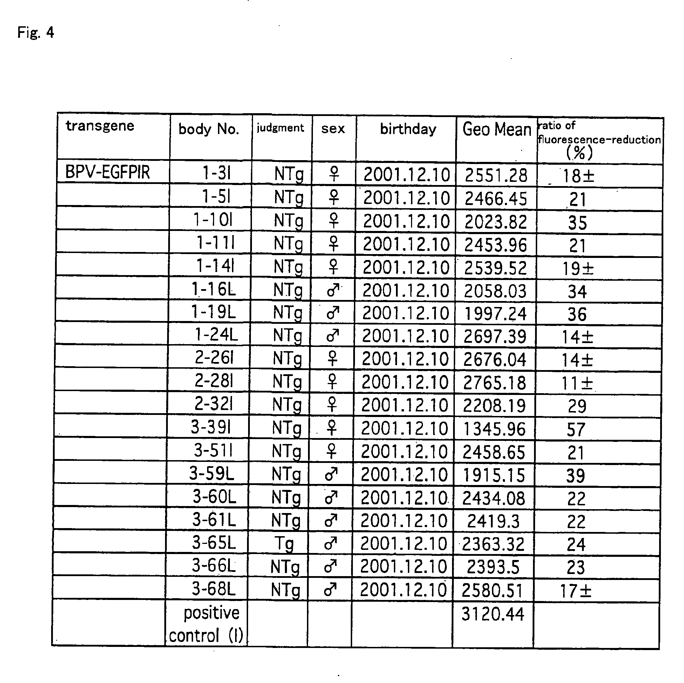

Using a heparinized capillary tube, blood was collected from the orbital venous plexus of a mouse that was 4 weeks old or older. Thereafter, using Lympholyte-M (manufactured by CEDARLANE), peripheral mononuclear cells were separated from the blood. Thereafter, fluorescence in the cells was analyzed with FACS (BD). As an analysis method, the mean value (GeoMean) of EGFP fluorescence in 5,000 lymphocytes was compared with the GeoMean mean value of a control mouse, so that the ratio of reduced fluorescence was calculated. As a result, with regard to BPV-HIR, the presence of lymphocytes with reduced EGFP fluorescence was confirmed in 27 ($\pm$6) out of the total 156 mice born (the ratio of obtaining mice with phenotype: 12.8% (16.6%)). On the other hand, with regard to BPV-EGFP IR, the presence of lymphocytes with reduced EGFP fluorescence was confirmed in 19 ($\pm$6) out of the total 71 mice born (the ratio of obtaining mice with phenotype: 18.3% (26.8%)) (Figures 3 and 4).

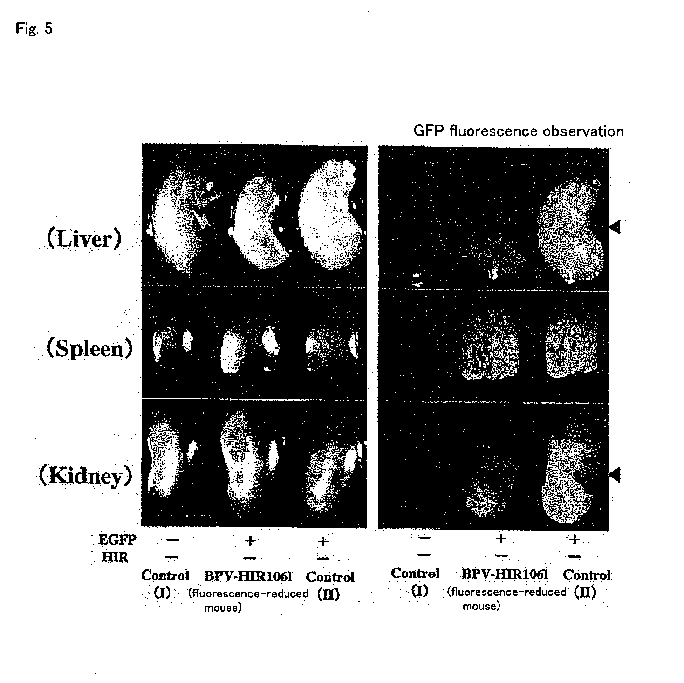

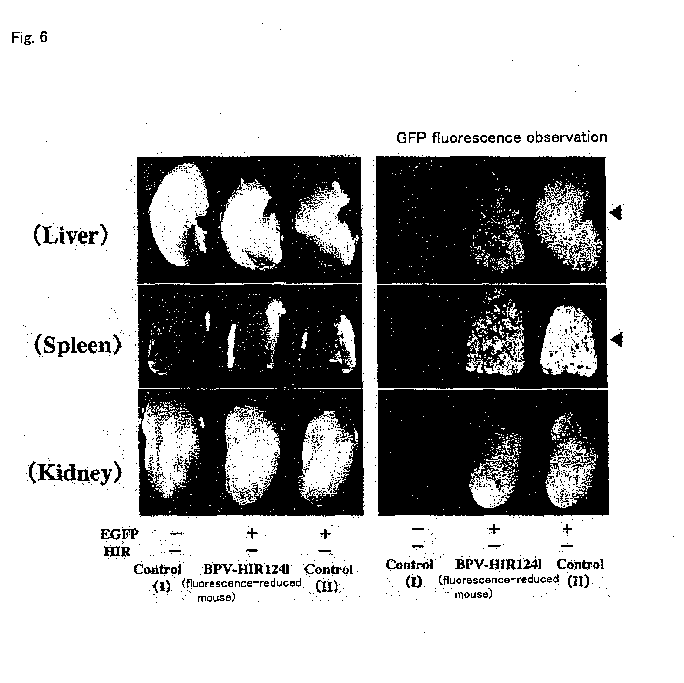

Mice that were 12 weeks old or older were prepared. From the mice, principal organs such as the brain, liver, heart, kidney, spleen, and thigh muscle were obtained. Using a fluorescence stereoscopic microscope (LEICA MZ FLIII; manufactured by Leica) to which a digital video camera (LEICA DC200; manufactured by Leica) was connected, EGFP fluorescence in those organs was observed. As a result, it was confirmed that EGFP fluorescence was significantly reduced in the liver, kidney, and spleen (Figures 5 and 6).

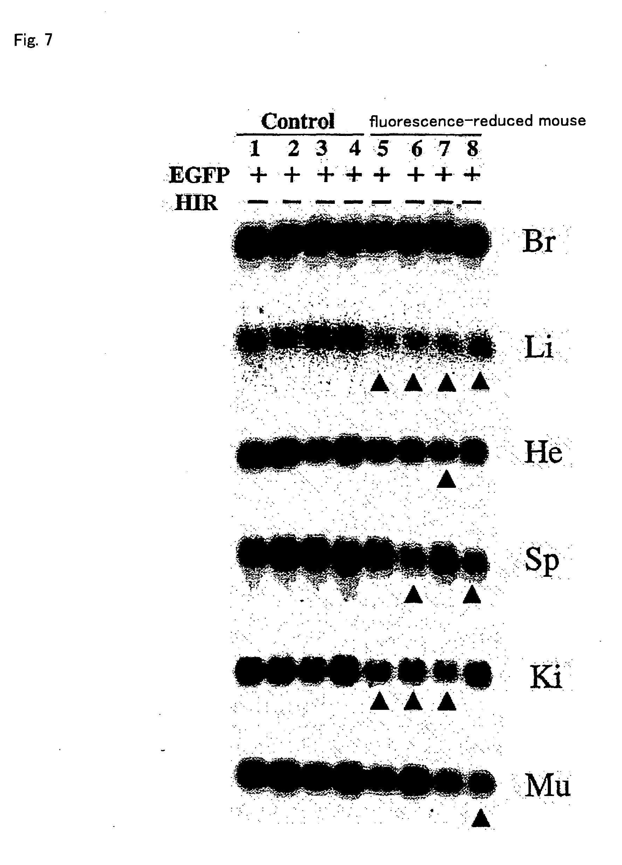

Subsequently, total RNA was extracted from the obtained organs using TRIzol (manufactured by Invitrogen). Approximately 10 µg of the total RNA was electrophoresed on 1% denatured agarose gel containing formalin. (In the case of the thigh muscle and heart, since EGFP is highly expressed in RNA, approximately 3 µg of the total RNA was used.) Thereafter, RNA was immobilized on a nylon filter (Hybond-XL; manufactured by Amersham Pharmacia), and this filter was then hybridized with a probe in a perfect hybridization solution (manufactured by Toyobo) for 2 hours. The resultant filter was washed with 2 x SSC and 0.1% SDS at 65°C for 5 minutes twice, and further washed therewith for 30 minutes twice. As a result of Northern hybridization, it was found that the expression of EGFP was significantly reduced in the liver, kidney, and spleen (Figure 7).

INDUSTRIAL APPLICABILITY

The method of the present invention enables the production of a non-human mammal with an RNAi phenotype more efficiently than the conventional methods for producing a non-human mammal with an RNAi phenotype. Moreover, in the analysis of genes associated with diseases or the analysis of genes that are targeted for medicaments by using the RNAi effect, it becomes possible to suppress genes more reliably than the use of the conventional mice, and thus, it is considered that the method of the present invention greatly contributes to the industry.

**Claims**

1. A method for producing a non-human mammal wherein the function of a target gene is suppressed, which comprises introducing into cells a papilloma virus vector into which the target gene has been incorporated.

2. The method according to claim 1 wherein the papilloma virus vector into which the inverted repeat sequence of the target gene has been introduced is used.

3. The method according to claim 1 or 2 wherein the papilloma virus is a bovine papilloma virus vector.

4. The method according to any of claims 1 to 3 wherein a papilloma virus vector into which a gene of interest has been incorporated is introduced into a fertilized egg, and a transgenic non-human mammal is produced by using the fertilized egg.

5. A non-human mammal wherein the function of a target gene is suppressed, which is produced by the method according to any of claims 1 to 4, a progeny thereof, or a portion thereof.

6. A recombinant vector formed by incorporating into a papilloma virus vector the inverted repeat sequence of a target gene capable of expressing in mammalian cells.

7. The recombinant vector according to claim 6 wherein the papilloma virus vector is a bovine papilloma virus vector.

8. The recombinant vector according to claim 6 or 7 which comprises the inverted repeat sequence of a target gene downstream of a promoter sequence capable of functioning in mammalian cells.

9. The recombinant vector according to any of claims 6 to 8 wherein the target gene is a gene of a foreign reporter protein or a mutant protein thereof.

10. The recombinant vector according to claim 9 wherein the foreign reporter protein is Enhanced green fluorescent protein (EGFP).

11. The recombinant vector according to any of claims 6 to 10 which is used to produce a transgenic non-human mammal.

12. A transformant having the recombinant vector according to any of claims 6 to 11.

13. An embryo generated from a fertilized egg derived from a non-human mammal into which the recombinant vector according to any of claims 6 to 11 has been introduced.

14. A fetus obtained by transplanting the embryo according to claim 13 into the uterus or oviduct of the corresponding non-human mammal, followed by development.

Fig. 1

Fig. 2

fluorescence-reduced mouse

Fig. 3

| transgene | body No. | judgment | sex | birthday | Geo Mean | ratio of fluorescence-reduction (%) |
|---|---|---|---|---|---|---|
| BPV-HIR | 1-8L | NTg | ♂ | 2001.9.6 | 1965.29 | 37 |
| | 2-20L | NTg | ♂ | 2001.10.15 | 1831.51 | 41 |
| | 2-33L | NTg | ♂ | 2001.10.15 | 2130.91 | 32 |
| | 4-67I | NTg | ♀ | 2001.11.20 | 2563.08 | 18± |
| | 4-71I | NTg | ♀ | 2001.11.20 | 2201.98 | 29 |
| | 5-73I | NTg | ♀ | 2001.11.26 | 2402.14 | 23 |
| | 5-74I | NTg | ♀ | 2001.11.26 | 2548.92 | 18± |
| | 5-85I | NTg | ♀ | 2001.11.26 | 2403.65 | 23 |
| | 5-89I | NTg | ♀ | 2001.11.26 | 2465.43 | 21 |
| | 5-91I | NTg | ♀ | 2001.11.26 | 2617.86 | 16± |
| | 5-98L | NTg | ♂ | 2001.11.26 | 2104.38 | 33 |
| | 5-99L | NTg | ♂ | 2001.11.26 | 1985.99 | 36 |
| | 5-100L | NTg | ♂ | 2001.11.26 | 2437.18 | 22 |
| | 6-102I | NTg | ♀ | 2001.11.28 | 2696.36 | 14± |
| | 6-106I | NTg | ♀ | 2001.11.28 | 2266.96 | 27 |
| | 6-124I | NTg | ♀ | 2001.11.28 | 2264.62 | 27 |
| | 6-128L | NTg | ♀ | 2001.11.28 | 2531.37 | 19± |
| | 6-133L | NTg | ♂ | 2001.11.28 | 1728.66 | 45 |
| | 6-134L | NTg | ♂ | 2001.11.28 | 2436.73 | 22 |
| | 6-137L | NTg | ♂ | 2001.11.28 | 2474.63 | 21 |
| | 6-138L | NTg | ♂ | 2001.11.28 | 2629.49 | 16± |
| | 6-139L | NTg | ♂ | 2001.11.28 | 2381.21 | 24 |
| | 6-142L | NTg | ♂ | 2001.11.28 | 2495.02 | 20 |
| | 6-144L | NTg | ♂ | 2001.11.28 | 2130.02 | 32 |
| | 6-145L | NTg | ♂ | 2001.11.28 | 2360.08 | 24 |
| | 6-146L | NTg | ♂ | 2001.11.28 | 2050.08 | 34 |
| | positive control (I) | | | | 3120.44 | |

Fig. 4

| transgene | body No. | judgment | sex | birthday | Geo Mean | ratio of fluorescence-reduction (%) |
|---|---|---|---|---|---|---|
| BPV-EGFPIR | 1-3I | NTg | ♀ | 2001.12.10 | 2551.28 | 18± |
| | 1-5I | NTg | ♀ | 2001.12.10 | 2466.45 | 21 |
| | 1-10I | NTg | ♀ | 2001.12.10 | 2023.82 | 35 |
| | 1-11I | NTg | ♀ | 2001.12.10 | 2453.96 | 21 |
| | 1-14I | NTg | ♀ | 2001.12.10 | 2539.52 | 19± |
| | 1-16L | NTg | ♂ | 2001.12.10 | 2058.03 | 34 |
| | 1-19L | NTg | ♂ | 2001.12.10 | 1997.24 | 36 |
| | 1-24L | NTg | ♂ | 2001.12.10 | 2697.39 | 14± |
| | 2-26I | NTg | ♀ | 2001.12.10 | 2676.04 | 14± |
| | 2-28I | NTg | ♀ | 2001.12.10 | 2765.18 | 11± |
| | 2-32I | NTg | ♀ | 2001.12.10 | 2208.19 | 29 |
| | 3-39I | NTg | ♀ | 2001.12.10 | 1345.96 | 57 |
| | 3-51I | NTg | ♀ | 2001.12.10 | 2458.65 | 21 |
| | 3-59L | NTg | ♂ | 2001.12.10 | 1915.15 | 39 |
| | 3-60L | NTg | ♂ | 2001.12.10 | 2434.08 | 22 |
| | 3-61L | NTg | ♂ | 2001.12.10 | 2419.3 | 22 |
| | 3-65L | Tg | ♂ | 2001.12.10 | 2363.32 | 24 |
| | 3-66L | NTg | ♂ | 2001.12.10 | 2393.5 | 23 |
| | 3-68L | NTg | ♂ | 2001.12.10 | 2580.51 | 17± |
| | positive control (I) | | | | 3120.44 | |

EP 1 541 681 A1

Fig. 5

GFP fluorescence observation

(Liver)

(Spleen)

(Kidney)

| EGFP | — | + | + | — | + | + |
| HIR | — | — | — | — | — | — |
| | Control (I) | BPV-HIR1061 (fluorescence-reduced mouse) | Control (II) | Control (I) | BPV-HIR1061 (fluorescence-reduced mouse) | Control (II) |

20

EP 1 541 681 A1

Fig. 6

GFP fluorescence observation

| (Liver) | | |
| (Spleen) | | |
| (Kidney) | | |

EGFP  —  +  +  —  +  +
HIR  —  —  —  —  —  —

Control (I)   BPV-HIR1241 (fluorescence-reduced mouse)   Control (II)   Control (I)   BPV-HIR1241 (fluorescence-reduced mouse)   Control (II)

Fig. 7

**INTERNATIONAL SEARCH REPORT**

International application No.

PCT/JP03/09155

A. CLASSIFICATION OF SUBJECT MATTER
   Int.Cl⁷ C12N15/09, C12N15/86, C12N5/10, A01K67/027, A01H5/00//
              (C12N5/10, C12R1:91)

According to International Patent Classification (IPC) or to both national classification and IPC

B. FIELDS SEARCHED

Minimum documentation searched (classification system followed by classification symbols)
   Int.Cl⁷ C12N15/00-15/90, C12N5/00-5/28, A01K67/027, A01H1/00-7/00

Documentation searched other than minimum documentation to the extent that such documents are included in the fields searched

Electronic data base consulted during the international search (name of data base and, where practicable, search terms used)
   SwissProt/PIR/GeneSeq, Genbank/EMBL/DDBJ/GeneSeq, WPI(STN),
   BIOSIS(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| P,Y | HASUWA H. et al., "Small interfering RNA and gene silencing in transgenic mice and rats.", FEBS Lett., Vol.532, No.1-2, 04 December, 2002 (04.12.02), pages 227 to 230 | 1-14 |
| P,Y | Andres Mannik et al., "Effective generation of transgenic mice by Bovine papillomavirus type 1 based self-replicating plasmid that is maintained as extrachromosomal genetic element in three generations of animals.", Plasmid, Vol.49, No.3, (May 2003), pages 193 to 204 | 1-14 |
| E,Y | WO 02/66638 A2 (Gen Com Co.), 29 August, 2002 (29.08.02), (Family: none) | 1-14 |

☒ Further documents are listed in the continuation of Box C.   ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 September, 2003 (12.09.03) | 30 September, 2003 (30.09.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No.

PCT/JP03/09155

| C (Continuation). DOCUMENTS CONSIDERED TO BE RELEVANT | | |
|---|---|---|
| Category* | Citation of document, with indication, where appropriate, of the relevant passages | Relevant to claim No. |
| A | Tatsuroh Joh et al., "Preparation of a murine cell line which stably expresses human T lymphotropic virus type I (HTLV-I), env. genome products.", Gene, Vol.161, No.2, (1995), pages 227 to 230 | 6-10 |
| A | Yuichiro OOE et al., "Papillomavirus Vector", Protein, Nucleic acid and Enzyme, Vol.40, No.17, (1995), pages 2539 to 2544 | 6-10 |

Form PCT/ISA/210 (continuation of second sheet) (July 1998)


SEQUENCE LISTING

<110> GenCom

<120> A method for producing non-human mammals having RNAi phenotype by using papillomavirus vector

<130> A31278A

<160> 4

<210> 1

<211> 33

<212> DNA

<213> Artificial Sequence

<220>

<223> Description of Artificial Sequence: Synthetic DNA

<400> 1

ggccatatag gccagttgta ctccagcttg tgc         33

<210> 2

<211> 33

<212> DNA

<213> Artificial Sequence

<220>

<223> Description of Artificial Sequence: Synthetic DNA

<400> 2

ggcctacatg gcctaaacgg ccacaagttc agc         33

<210> 3

<211> 27

<212> DNA

<213> Artificial Sequence

<220>

<223> Description of Artificial Sequence: Synthetic DNA

<400> 3

cggaccgtaa acggccacaa gttcagc                    27


<210> 4

<211> 27

<212> DNA

<213> Artificial Sequence

<220>

<223> Description of Artificial Sequence: Synthetic DNA

<400> 4

cggtccgagt tgtactccag cttgtgc                    27